Europäisches Patentamt

European Patent Office

Office européen des brevets

(11) Publication number: **0 001 681**
**A1**

# EUROPEAN PATENT APPLICATION

(21) Application number: **78300397.3**

(22) Date of filing: **19.09.78**

(51) Int. Cl.²: **C 07 C 29/28**
**C 07 C 31/08, C 07 C 31/10**

(30) Priority: **06.10.77 GB 41527/77**

(43) Date of publication of application:
**02.05.79 Bulletin 79/9**

(84) Designated contracting states:
**DE FR GB**

(71) Applicant: **BP Chemicals Limited**
**Britannic House Moor Lane**
**London, EC2Y 9BU(GB)**

(72) Inventor: **Cane, Charles**
**BP Chemicals Limited Saltend Hedon**
**Hull North Humberside(GB)**

(74) Representative: **Harry, John et al,**
**BP TRADING LIMITED Patents & Licensing Division**
**Chertsey Road**
**Sunbury-on-Thames Middlesex TW16 7LN(GB)**

(54) Dehydration of alcohols, preferably ethanol, using a cyclohexane entrainer.

(57) The present invention is a continuous process for producing substantially anhydrous alcohols from aqueous solutions by distilling the aqueous alcohol with cyclohexane in a drying column withdrawing a ternary azeotrope overhead and recovering substantially anhydrous alcohol containing not more than 0.1% by weight of water from the base of the drying column. The overhead distillate is fed into a decanter maintained at a temperature below the boiling point of the distillate for at least 10 minutes to separate an organic hydrocarbon phase from an aqueous phase, and the organic hydrocarbon phase is recycled to the top of the drying column. The aqueous phase is fed into the lower half of a recovery column and an overhead distillate product containing predominantly organic compounds from the recovery column is recycled to the drying column. The invention not only obviates the use of toxic entrainers such as benzenes but dramatically reduces phase separation time in the decanter. Alcohols which may be dried by this process include ethyl alcohol.

EP 0 001 681 A1

Croydon Printing Company Ltd.

## DEHYDRATION OF ETHANOL USING CYCLOHEXANE ENTRAINER

The present invention relates to improvements in processes for the removal of water from aqueous alcohols, preferably from alcohols which form azeotropic mixtures with water using cyclohexane as entrainer.

Various methods have been suggested for drying alcohols whether obtained from fermentation or synthetic processess. One such method is azeotropic distillation using an entrainer. Several entrainers have been used for this purpose including, glycols and hydrocarbon solvents. Of the hydrocarbon solvents benzene has hitherto been the most widely used. Benzene, however, suffers from several disadvantages. Firstly, the toxicity of benzene has rendered it unacceptable especially if such alcohols are to be used as potable spirits. Secondly, the rate of phase separation of azeotropic distillates containing benzene is very slow. This latter drawback has been overcome by using n-heptane in conjunction with benzene to form a quaternary azeotrope. However, such quaternary azeotropes containing n-heptane not only fail to resolve the problem of toxicity but also take overhead a smaller amount of water relative to benzene thus reducing the efficiency of the drying process. An additional problem has also been noted with the separation of the aqueous and organic phases from the distillate fed into a decanter. In some instances, in spite of using high boil up ratios (boil-up ratio is the amount of azeotrope product taken overhead to the amount of dried final product removed from the base of the drying

1

2                           0001681

still) the apparent rapid initial separation of the phases is deceptive in that the organic layer still contains a significant amount of water. This inefficiency becomes apparent if the organic phase recovered from the initial separation is allowed to stand in a cool environment for a few days when a further aqueous layer separates. Thus, substantially complete separation has only been achieved by allowing prolonged periods for separation of the phases. One example of such an entrainer is benzene but even in this case substantially complete separation has not been achieved in spite of allowing several hours for phase separation.

It has now been found that by using cyclohexane as an entrainer under specific distillation-conditions, the toxicity problems may be minimised and substantially complete separation of the aqueous phase may be achieved in a relatively short time.

Accordingly, the present invention is a continuous process for producing substantially anhydrous alcohols from aqueous solutions thereof comprising distilling a mixture of aqueous alcohol and cyclohexane fed to the upper half of a drying column withdrawing a ternary azeotrope as an overhead distillate and continuously recovering substantially anhydrous alcohol containing not more than 0.1% by weight of water from the base of the drying column, feeding the distillate into a decanter maintained at a temperature below the boiling point of the distillate for at least 10 minutes to separate an organic hydrocarbon phase from an aqueous phase, recycling the organic hydrocarbon phase to the top of the drying column, feeding the aqueous phase containing at least 30% by weight of the feed to the drying column into the lower half of a recovery column, recycling an overhead distillate product containing predominantly organic compounds from the recovery column to the drying column to a point intermediate between the aqueous ethanol feed point and the top of the drying column, and removing water substantially free from organics from the base of the recovery column.

The alcohols which may be dried by the process of the present

2

0001681

invention are suitably saturated aliphatic alcohols containing 2 or 3 carbon atoms and is preferably ethyl alcohol.

The feed to the drying column is a heterogeneous mixture of aqueous alcohol and cyclohexane. This mixture suitably contains between 80 and 90% by weight of alcohol, between 3 and 10% by weight of water and between 5 and 10% of cyclohexane.

The drying column suitably contains more than 50 plates, preferably between 55 and 70 plates. The heterogeneous mixture of aqueous alcohol and cyclohexane is suitably fed into the drying column at a point between the 35th and 45th plate from the base or kettle of the column.

Substantially anhydrous ethanol containing not more than 0.1% and preferably not more than 0.05% by weight of water is continually drawn from the base of the drying column.

The ternary azeotropic distillate withdrawn overhead from the drying column suitably contains over 70% by weight of cyclohexane, between 15 and 20% by weight of alcohol and between 2 and 7% by weight of water. This azeotropic distillate is then fed into a decanter which is maintained at a temperature below the boiling point of the ternary azeotrope. If the alcohol is ethanol, the decanter is suitably maintained below $60^{\circ}$C preferably between 35 and $50^{\circ}$C. The use of cyclohexane as an entrainer enables rapid separation of the organic hydrocarbon phase from the aqueous phase in the decanter. For example, in the case of a ternary mixture containing ethanol, water and cyclohexane, the aqueous phase separation commences immediately on starting the settling period and is rapidly completed. To achieve substantially complete separation between the aqueous and organic phases, the residence time of the overhead product from the distillation in the decanter is at least 10 minutes preferably between 12 and 120 minutes.

On separation of these phases in the decanter, the organic hydrocarbon phase contains for instance between 10 and 15% by weight of the alcohol in the case of ethanol and this phase is recycled to the top of the drying column.

The aqueous phase from the decanter which contains more than 30% by weight of the feed to the drying column is fed to the lower part of the recovery column. The recovery column suitably contains between 30 and 40 plates. The aqueous phase from the decanter is preferably fed to a point between the 7th and 20th plate from the base of the column or the kettle. In this column, the organic content of the aqueous phase is distilled as overheads preferably using a reflux ratio which is in the range of between 5:1 and 10:1. The overhead distillate from the recovery column suitably containing over 70% alcohol and between 15 and 30% of the cyclohexane entrainer is recycled to be mixed with the feed to the drying column. Water, substantially free from organics, is removed from the base of the recovery column which may be discharged at waste.

The invention is further illustrated with reference to the following examples.

Comparative Test not according to the invention

Ethanol:water azeotrope (13.6 kg containing 0.69 kg of water) and cyclohexane (1 kg) were continuously fed over 31 hours onto the 40th plate of a 58 plate Oldershaw column (50 mm diameter) drying still column. Ethanol:cyclohexane:water ternary azeotrope (61.6 kg containing 4.6% w/w of water) was taken overhead, condensed and the two phases which formed were separated in a decanter at 38°C. The residence time in the decanter of the total overhead product and the separated upper phase amounted to 6 minutes and 4 minutes respectively. Organic upper phase (56.3 kg, containing 3.8% w/w of water) was recycled from the decanter to the top of the drying column. This shows that the separation of water phase was not substantially complete.

Dry ethanol final product (9.4 kg, containing 0.05% w/w water and 1 ppm cyclohexane) was collected from the base of the drying column. The boil up ratio (viz 61.6 over 9.4) was 6.6:1.

Aqueous phase (5.3 kg) from the drying column decanter was fed onto the 10th plate of a 35 plate Oldershaw column (50 mm diameter) recovery still. Ethanol:cyclohexane:water azeotrope mixture (4.7 kg) was taken overhead using a reflux ratio of 7:1 and this was recycled and mixed with the feed to the drying column.

Water (0.6 kg) was removed from the base of the recovery column.

Example

Ethanol:water azeotrope (4.8 kg containing 0.3 kg water) and cyclohexane (0.75 kg) were fed over 13 hours onto the 40th plate of a 58-plate Oldershaw column (50 mm diameter) drying still.

Ethanol:cyclohexane:water ternary azeotrope (12.5 kg containing 4.6% w/w water) was taken overhead, condensed and the two phases which formed were separated in a horizontal, cylindrical decanter at $15^{\circ}$C. The residence time in the decanter of the total overhead product and the separated upper phase amounted to ca. 27 and 19 minutes respectively. Organic phase (8.7 kg) from the decanter, containing 0.2% w/w of water, was recycled to the top of the drying column.

Dry ethanol final product (4.6 kg containing 0.07% w/w water) was collected from the base of the drying column. The boil up ratio (viz 12.5 over 4.6) was 2.7:1.

Aqueous phase (3.8 kg) from the drying column decanter was fed onto the 10th plate of a 35 plate Oldershaw column (50 mm diameter) recovery still. Ethanol:cyclohexane:water azeotrope mixture (3.5 kg) was taken overhead with a reflux ratio of 7:1 and this was recycled and mixed with the feed to the drying still. Water (0.3 kg) was removed from the base of the recovery column.

Thus, a virtually complete removal of water was achieved as above by use of a relatively low boil-up rate of 2.7:1 (of final product) compared with a boil-up rate of 6.6:1 which was required in Example 2(a). Thus, the energy consumption required for such ethanol drying may be virtually halved.

1. A continuous process for producing substantially anhydrous alcohols from aqueous solutions thereof comprising distilling a mixture of aqueous alcohol and cyclohexane fed to the upper half of a drying column, withdrawing a ternary azeotrope as an overhead distillate and continuously recovering substantially anhydrous alcohol containing not more than 0.1% by weight of water from the base of the drying column, feeding the distillate into a decanter maintained at a temperature below the boiling point of the distillate for at least 10 minutes, to separate an organic hydrocarbon phase from an aqueous phase, recycling the organic hydrocarbon phase to the top of the drying column, feeding the aqueous phase containing at least 30% by weight of the feed to the drying column into the lower half of a recovery column recycling an overhead distillate product containing predominantly organic compounds from the recovery column to the drying column to a point intermediate between the aqueous ethanol feed point and the top of the drying column, and removing water substantially free from organics from the base of the recovery column.

2. A process according to claim 1 wherein the alcohols which are dried are saturated aliphatic alcohols containing two or three carbon atoms.

3. A process according to claim 2 wherein the alcohol is ethyl alcohol.

4. A process according to any of the preceding claims wherein the feed to the drying column contains between 80 and 90% by weight of alcohol, between 3 and 10% by weight of water and between 5 and 10% by weight of cyclohexane.

5.  A process according to any of the preceding claims wherein the drying column contains more than 50 plates.

6.  A process according to claim 5 wherein the drying column contains between 55 and 70 plates.

7.  A process according to any of the preceding claims wherein the mixture of aqueous alcohol and cyclohexane is fed into the drying column at a point between the 35th and 45th plate from the base of the column.

8.  A process according to any of the preceding claims wherein the azeotropci distillate withdrawn overhead from the drying column contains over 70% by weight of cyclohexane, between 15 and 20% by weight of alcohol and between 2 and 7% by weight of water.

9.  A process according to any of the preceding claims wherein the decanter is maintained below $60^{\circ}$C.

10.  A process according to claim 9 wherein the decanter is maintained between 35 and $50^{\circ}$C.

11.  A process according to any of the preceding claims wherein the residence time of the distillate in the decanter is between 12 and 120 minutes.

12.  A process according to any of the preceding claims wherein the recovery column contains between 30 and 40 plates.

13.  A process according to any of the preceding claims wherein the aqueous phase from the decanter is fed to the recovery column at a point between the 7th and 20th plate from the base of the column.

14.  A process according to any of the preceding claims wherein the organic content of the aqueous phase is distilled as overheads in the recovery column using a reflux ratio in the range of between 5:1 and 10:1.

15.  A process according to any of the preceding claims wherein the overhead distillate from the recovery column containing over 70% alcohol and between 15 and 30% of cyclohexane is recycled to be mixed with the feed to the drying column.

16.  A process according to any of the preceding claims wherein substantially anhydrous ethanol containing not more than 0.05% by weight of water is continually withdrawn from the base of the

drying column.

A process for producing substantially anhydrous ethanol according to claim 1 as hereinbefore described with reference to the Examples.

# EUROPEAN SEARCH REPORT

European Patent Office

## DOCUMENTS CONSIDERED TO BE RELEVANT

| Category | Citation of document with indication, where appropriate, of relevant passages | Relevant to claim |
|---|---|---|
| - | <u>DE - B - 1 014 974</u> (DISTILLERS)<br>* claims 1, 2, 3, 9 *<br><br>-- | 1,2 |
| - | <u>DE - B - 1 219 011</u> (BASF)<br>* example *<br><br>-- | 5 |
| A | Chemical Abstracts vol. 46, no. 19, 1952,<br>Columbus, Ohio, USA<br>K. ZIEBORAK "Azeotropic properties of systems ethanol-water-hydrocarbons"<br>column 8947 f-g<br>& Prace Glownego Inst. Chem. Przemysl no. 1, 1951 page 1 to 44<br><br>-- | |
| A | Chemical Abstracts vol. 85, no. 3, 1976,<br>Columbus, Ohio, USA<br>K.U. RIEDEL et al "Method for the purification of alcohol with cyclohexane"<br>page 501, column 1, abstract no.18983n<br>& Branntweinwirtschaft vol. 116, no. 5, 1976, page 69-70<br><br>---- | |

### CLASSIFICATION OF THE APPLICATION (Int. Cl.)

C 07 C  29/28
C 07 C  31/08
C 07 C  31/10

### TECHNICAL FIELDS SEARCHED (Int.Cl.)

C 07 C  27/30
C 07 C  29/28
C 07 C  31/08
C 07 C  31/10

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| Berlin | 18-12-1978 | KNAACK |

EPO Form 1503.1  06.78